# EUROPEAN PATENT APPLICATION

(11) **EP 4 792 874 A1**
(43) Date of publication of application: **19.08.2026**
(21) Application number: 24877224.6
(22) Date of filing: 10.10.2024
(51) Int. Cl.: A61N 5/06, A61N 5/067

(54) **DEVICE FOR TREATING COUGHING**

(30) Priority: 13.10.2023 JP 2023177199
(71) Applicant: Teijin Pharma Limited, Tokyo 100-0013 (JP)
(72) Inventor: NANJO Takuya, Tokyo 100-0013 (JP); MOTOHASHI Yuto, Tokyo 100-0013 (JP); KAWASE Yuki, Tokyo 100-0013 (JP); ISHIBASHI Naoya, Tokyo 100-0013 (JP)
(74) Representative: Hoffmann Eitle
(86) International application number: PCT/JP2024/036231
(87) International publication number: WO 2025/079630

(57) **Abstract**

The cough treatment device has a light beam source emitting a light beam and a light beam irradiation probe performing irradiation with the light beam, wherein light beam irradiation is performed percutaneously with the light beam emitted by the light beam source from the light beam irradiation probe toward a patient's cervical vagus nerve and the vicinity thereof. Instead of drug therapy that causes systemic symptoms, this device directly suppresses hyperactivity of the vagus nerve near the airway, which is the cause of cough, and achieves a less-adverse-effect, safe, and effective treatment method.

## Description

### [Technical Field]

The present invention relates to a cough treatment device that treats cough by irradiating the vagus nerve with a light beam.

### [Background Art]

Cough is originally a defense mechanism to expel secretions and foreign substances that have accumulated in the airway. Cough is classified based on the duration as acute (less than 3 weeks), persistent (3 or more and less than 8 weeks), or chronic (8 weeks or more). The most common cause of acute cough is a viral common cold, and most of the patients will recover by treating the cause. Persistent or chronic coughs are classified into dry coughs without sputum and wet coughs with sputum based on whether sputum is produced. Most coughs are dry coughs in which the cough itself is the target of treatment. Incidentally, wet cough is caused by excessive secretion of mucus in the airway.

A cough that lasts for eight weeks or more is called a chronic cough, the prevalence of which in Japan is reportedly approximately 2-4% (approximately 2.5-5 million people). Chronic cough is diagnosed and treated in accordance with a flow in the guidelines, and in many cases, effective is a treatment for each cause of the underlying disease, such as cough variant asthma, atopic cough, gastroesophageal reflux disease, post-infectious cough, or sinobronchial syndrome. However, there are intractable cases in which even a treatment for the underlying disease is not effective.

The intractable cases of chronic cough reportedly account for approximately 20% (approximately 200,000-400,000 people) of patients receiving treatment for chronic cough in Japan. Furthermore, in recent years, the concept of cough hypersensitivity syndrome (CHS) has been proposed to describe a common pathological conditions for treatment-resistant cough, regardless of the existence or non-existence of an underlying disease, which is drawing attention to intractable chronic cough.

Centrally acting antitussives containing codeine or dihydrocodeine are sometimes used as a therapeutic drug for cough. Recently, a selective P2X3 receptor antagonist (gefapixant citrate) was approved in Japan as a therapeutic drug for intractable chronic cough. However, cough treatment with therapeutic drugs has been reported to cause adverse effects by their systemic actions such as constipation, nausea, vomiting, dizziness, and taste disturbance (NPL 1), and pharmaceuticals with reduced adverse effects are being newly developed (PTL 1).

Low level laser therapy (LLLT), a phototherapy, is a physical treatment method that has anti-inflammatory action and suppresses nerve hyperactivity, the application of which is considered for various diseases such as overactive bladder, irritable bowel syndrome, chronic pain, and facial neuralgia (PTL 2).

### [Citation List]

### [Patent Literature]

[PTL 1] Japanese Patent No. 6725188
[PTL 2] WO2022/019293

### [Non-patent literature]

[NPL 1] Lyfnua Tablets 45 mg package insert (MSD, Kyorin)

### [Summary of Invention]

### [Technical Problem]

Although chronic cough is not a life-threatening disease, the chronic cough can cause a decline in performance both in public and private matters, resulting in a decline in the patient's quality of life. As mentioned above, the therapeutic drugs, though available, have a problem of adverse effects, and thus the current therapeutic or preventive effects cannot be said to be sufficient. On the above background, new treatments or preventive methods for chronic cough are desired.

Selective P2X3 receptor antagonists, which are being developed as a therapeutic drug for intractable or cryptogenic chronic cough, inhibit the binding of extracellular adenosine triphosphate (ATP) to ATP receptors expressed on vagal C-fibers in the airway, and thus can suppress cough elicitation associated with airway inflammation. P2X3 receptors are specifically expressed in C-fibers of afferent nerves (sensory nerves) and are densely distributed in the internal organs, skin, joints, etc. Therefore, oral administration of such therapeutic drugs inhibits systemic P2X3 receptors other than those in the airway, causing adverse effects.

The present invention provides a cough treatment device that directly suppresses cough-causing hyperactivity of the vagus nerve near the airway, instead of drug therapy that causes systemic symptoms, thereby achieving a less-adverse-effect, safe, and effective treatment method.

### [Solution to Problem]

The present invention provides a phototherapy device, as described below, that suppresses cough by applying a light beam such as a low-power laser to the vagus nerve.
(1) A cough treatment device, comprising: a light beam source emitting a light beam and a light beam irradiation probe performing irradiation with the light beam, light beam irradiation being performed percutaneously with the light beam emitted by the light beam source from the light beam irradiation probe toward a patient's cervical vagus nerve and the vicinity thereof.
(2) The cough treatment device according to (1), wherein the light beam irradiation probe is a device that irradiates an inferior ganglion, superior ganglion, pharyngeal branch, or superior laryngeal nerve of a cervical vagus nerve.
(3) The cough treatment device according to (1), wherein the light beam irradiation probe is a device that irradiates the patient's neck behind an angle of mandible or below a mastoid process.
(4) A cough treatment device according to (1), wherein an average power of a light beam of irradiation from the light beam irradiation probe is 200 mW to 8 W, an average power density obtained by dividing an average power of a light beam of irradiation from the light beam irradiation probe by the irradiation area of the light beam is 250 mW/cm² or more to 10,000 mW/cm² , an energy of the amount of light beam irradiation from the light beam irradiation probe is 60 J to 4,800 J per treatment, an energy density of the amount of light beam irradiation from the light beam irradiation probe divided by the irradiation area of the light beam is 75 J/cm² to 6,000 J/cm² per treatment, and a wavelength of a light beam of irradiation from the light beam irradiation probe is 700 nm to 900 nm.

### [Advantageous Effects of Invention]

The cough treatment device of the present invention percutaneously irradiates with a low-power laser near the cervical vagus nerve, and thereby exhibits the effect of reducing the number of coughs, similar to that by the centrally acting antitussive drug of dihydrocodeine, and can be used clinically as a new treatment method without the adverse effects such as constipation, vomiting, and taste disturbance seen in drug therapy.

### [Brief Description of Drawings]

Fig. 1 is a schematic diagram of an embodiment of a phototherapy device of the present invention.
Fig. 2 is a schematic diagram of an embodiment of a phototherapy device of the present invention.
Fig. 3 shows the therapeutic effect on cough.

### [Description of Embodiments]

The vagus nerve, one of the cranial nerves, emerges from the medulla oblongata and is widely distributed to the external auditory canal, auricle, pharynx, larynx, intrathoracic organs such as the trachea, bronchi, lungs, heart, and esophagus, and intraabdominal organs such as the stomach, intestines, liver, pancreas, spleen, and kidneys. The glossopharyngeal nerve, another cranial nerve, provides sensation from the throat and the posterior third of the tongue to the ear. These cranial nerves are roughly divided into efferent fibers that transmit information from the brain to peripheral organs and control movement, and afferent fibers of sensory nerves that transmit information from peripheral organs to the brain. Information sensed by each organ is transmitted to the brain via the afferent vagus nerve, and efferent fibers act to be involved in various physical activities, such as swallowing, coughing, and vomiting.

The vagus nerve extends from the medulla oblongata, comes out of the cranial cavity, runs vertically along the carotid arteries in both sides of the neck, branches off to the laryngopharynx and lungs, and then the pharyngeal branch and superior laryngeal nerve join together, which branch off to the pharynx and larynx at the inferior ganglion of a collection of nerve cell bodies below the auricle.

Cough receptors are distributed between and beneath the tracheal epithelial cells, and when various stimuli such as foreign substances or inflammation occur in these cells, the vagus nerve fires, the signal of which is transmitted to the medullary cough center to cause a cough reflex. As a result, the diaphragm and thoracic tissues move rapidly to cause cough, which allows expulsion of foreign substances and secretions.

Chronic cough in which a cough prolongs after curing a cold or viral infection, having various pathological conditions, is mostly caused by increased reactivity of the peripheral nervous system, or airways, which are involved in inducing cough. In particular, in pathological conditions of increased sensitivity of cough receptors, activation of C fibers, which are unmyelinated, thin, and constituting slow-transmitting sensory nerves, occurs as an increased cough reflex.

The phototherapy device of the present invention treats patients with cough by irradiating with a predetermined light beam upstream of the afferent nerve where cough receptors are located, thereby suppressing increased activation of the vagus nerve and suppressing the cough reflex.

### Phototherapy device

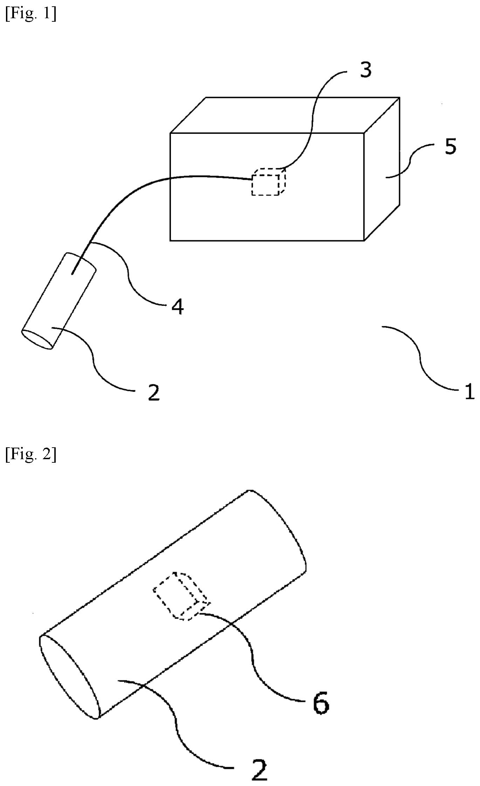

An embodiment of a light beam irradiation device 1 of the present invention will be described with reference to the drawings. As shown in Fig. 1, the light beam irradiation device 1 of the present embodiment is a medical device for treating or preventing cough by performing percutaneous irradiation on the vagus nerve running through the patient's neck and the vicinity thereof. The light beam irradiation device is equipped with a light beam irradiation probe 2 for irradiation of a light beam, a light beam source 3, a probe cable 4 for connecting the light beam source and the light beam irradiation probe, and a main body 5 having the light beam source built in.

The patient performs treatment or prevention by applying the light beam irradiation probe 2 on the skin just above the inferior ganglion of the vagus nerve in the neck and performing irradiation with a light beam. Fig. 1 shows a situation in which a light beam is guided by a probe cable 4, and the main body is provided with a power supply (not shown). As shown in Fig. 2, there can be adopted a configuration in which the light beam irradiation probe 2 has a light beam source and a power supply 6 built-in, which allows the patient to carry the device around and thus to perform treatment or prevention even when going out. In these diseases, many of the patients are not hospitalized and have their daily lives like healthy people, and thus a portable treatment or prevention device that is available when necessary is convenient.

In order to enhance the safety for irradiation with a light beam, there is preferably provided a structure which prevents the diffuse reflected light from the light-beam irradiated skin from leaking outside when the tip of the light beam irradiation probe comes into contact with the skin. There is more preferably provided a structure in which a sensor for detection of contact with the skin and a control calculation unit are equipped at the tip and the vicinity of the light beam irradiation probe to enable irradiation only when the light beam irradiation probe is correctly in contact with the skin of the irradiation area.

Adverse effects to the light beam include a burn attributable to an increase in the temperature of the skin. The burn can be prevented by a method which reduces an increase in the skin temperature by a pulse irradiation of intermittently irradiating with a light beam maintaining the energy that is the amount of light beam and the energy density. In that case, the repetition frequency is preferably 0.5 to 10 Hz. Further, there is preferably provided a structure in which the temperature is lowered by forced convection using a fan, a compressor or the like as an air blowing source to blow air to the skin through a flow path or the like.

### Light beam irradiation area

The light beam irradiation area in the present invention is the vagus nerve in the patient's neck and the vicinity thereof, wherein the light is applied percutaneously to such an area. Specifically, the radiation is applied percutaneously to the inferior ganglion, superior ganglion, pharyngeal branch or superior laryngeal nerve branching from the vagus nerve, or glossopharyngeal nerve running through the vicinity thereof in the vagus nerves in the neck. The ideal irradiation area is the vicinity of the inferior ganglion a little downstream the point where the branching vagus nerves joins together. However, since the above nerves ascend parallel before joining to the vagus nerve at the inferior ganglion, it is sufficient for the light beam to hit the vicinity area of the inferior ganglion. Irradiation is preferably performed toward the inferior ganglion where the nerves branching from the vagus nerve join together, or toward the vagus nerve, laryngeal branch, superior laryngeal nerve, and glossopharyngeal nerve together.

The specific area in the neck where this nerve is running is the area near to the top of the head in the vagus nerve running vertically through the lateral region of the neck, and when viewed from the front side of the body, the area behind the angle of the mandible, below the mastoid process, or between the two.

### Light beam for irradiation

The light beam irradiation device of the present invention preferably has one or multiple following light beam conditions in order to exert a therapeutic effect on cough. That is, the conditions are as follows: the average power is 200 mW or more, the average power density (average power/irradiation area of light beam) is 75 mW/cm² or more, the amount of light beam in terms of energy is 130 J or more per treatment, and the energy density (amount of light beam in terms of energy/irradiation area of light beam) is 45 J/cm² or more per treatment or prevention, and the wavelength is 700 to 900 nm. Among these conditions, the average power, average power density, energy, and energy density are obtained by converting the conditions of the light beam in the test performed by using the cough model animals to the assumed-clinical conditions based on the light beam transmissiveness obtained by simulation. Further, the light beam for irradiation may be a single irradiation by continuous irradiation or an intermittent irradiation which repeats start and stop of irradiation.

The light beam irradiation device of the present invention may be set to preferable light beam conditions and perform irradiation in the treatment or prevention of cough, or the light beam irradiation device is provided with a light beam source capable of performing irradiation under the predetermined preferable light beam conditions.

A laser beam excited by a semiconductor device etc. is an example of a light beam satisfying the irradiation conditions of the present invention, such as average power, average power density, energy, energy density, and wavelength. Alternatively, LED light beam may be used that meets the requirements of the present invention. As reported in Photochemical & Photobiological Sciences 2018;17(8):1003-1017, LED light beam as well as laser beam is widely used as light beam for treatment or prevention.

### Average power range

As shown in Journal of Clinical Laser Medicine & Surgery, 1991;9(4):267-75, the relationship between the amount of light beam and the effect is explained by the Arndt-Schuwltz law. The Arndt-Schuwltz law explains that when the amount of light beam exceeds a certain threshold, biological activity is promoted, and when the amount of light beam is further increased, biological activity is suppressed. The conditions indicating the amount of light beam in the present invention are peak power, average power, average power density, energy, and energy density. Therefore, a condition which has a value greater than the lower limit value confirmed to be effective will presumably exhibit a similar neurotransmission inhibitory effect.

As described later in the Example, attempt was made to convert the experimental conditions of the nonclinical tests to those equivalent to the clinical tests by examining the transmitted amount of the light using light scattering simulation by Monte Carlo Modeling of Light Transport in Multi-layered Tissues (hereinafter referred to as MCML) using Monte Carlo method (Computer Methods and Programs in Biomedicine, Volume 47, Issue 2, July 1995, Pages 131 -146), described in WO2022/019293 (PTL 2). The conditions such as peak power, average power, average power density, energy, and energy density related to the amount of light beam in the guinea pigs can be converted to the lower limits of human equivalent conditions by multiplying each condition parameter by 0.3. Therefore, the average power in the present invention is 0.2 W (200 mW) or more, preferably 0.2 W (200 mW) to 8 W, and more preferably 0.2 W (200 mW) to 0.8 W (800 mW).

### Range of average power density

In the present invention, the average power density (average power per unit area) is 250 mW/cm² or more, more preferably 250 to 10,000 mW/cm², and more preferably 250 to 1,000 mW/cm².

### Energy range

The amount of light beam in terms of energy in the present invention is 60 J or more per treatment, preferably 60 to 4,800 J, more preferably 60 to 2,400 J, and more preferably 240 to 2,400.

### Range of energy density

The amount of light beam in terms of energy density in the present invention is 75 J/cm² or more per treatment, preferably 75 to 6,000 J/cm², more preferably 75 to 3,000 J/cm², and more preferably 300 to 3,000 J/cm².

### Wavelength range

The wavelength of 808 nm used in the effectiveness test of the examples is included in the near infrared region of 700 to 900 nm. As shown in Journal of Physics D: Applied Physics, 2005, 38, 2543-2555, the transmissiveness to the living body is the same in the near infrared region, and thus the same effectiveness exists in the range of 700 to 900 nm. Further, the absorption spectrum of cytochrome C oxidase, which has been reported to be involved in the mechanism of action in phototherapy, is reported to be the same in the range of 780 to 850 nm as shown in Journal of Biological Chemistry, 2005; 280(6): 4761-4771. From the above, the wavelength in the present invention is preferably 700 to 900 nm, more preferably 780 to 850 nm, and even more preferably 788 to 828 nm.

### Irradiation embodiment

As an embodiment, treatment or prevention is preferably performed at a frequency of twice a day to once a week or so. It can also be performed depending on the physical condition such as when the symptom develops or, is likely to develop. The treatment time in terms of the duration of light beam irradiation is preferably approximately 3 to 60 minutes. In this case, a long continuous laser beam irradiation may cause a burn on the skin. A high-output irradiation can be performed by repeating intermittent irradiation such as 1-minute irradiation and 10-second stop, or 30-second irradiation and 5-second stop. In that case, the surface area of the irradiation area is approximately 0.5 to approximately 6 cm², preferably 0.6 to 3 cm², and the shape is preferably circular, elliptical, rectangular, or the like.

### Experiment Example

### [Example 1] Examination of light beam transmissiveness by simulation

The effectiveness of phototherapy depends on the amount of light at the target tissue. However, once a light beam irradiates the living body, it is repeatedly scattered and absorbed, and the amount of light decreases exponentially depending on the distance from the skin surface (Lambert-Beer law); thus, the effectiveness in deep tissues needs to be estimated considering the transmission of light beam. Therefore, the optimal output conditions are effectively calculated by comparing the distance to the nerves of animals used in non-clinical studies with that of humans. For example, the distance from the skin surface to the vagus nerve in the neck of a guinea pig used as a cough model animal was approximately 15 mm. In contrast, ultrasound echography image of the location of the vagus nerve in the human neck revealed the vagus nerve to be located at a depth of approximately 10 to 15 mm. Therefore, since the vagus nerve may be located deeper in guinea pigs than in humans, the irradiation conditions equivalent to clinical use may be lower than that for experiment in non-clinical tests. Incidentally, when the skins are compared between rodents and humans, though the structure is different, the constituents are the same, and thus the light beam transmissiveness presumably depends on the distance from the skin surface to the target tissue rather than on the difference of the animal species.

Next, we attempted to convert the experimental conditions of the nonclinical tests to those equivalent to the clinical tests by examining the transmitted amount of the light using light scattering simulation by Monte Carlo Modeling of Light Transport in Multi-layered Tissues (hereinafter referred to as MCML) using Monte Carlo method (Computer Methods and Programs in Biomedicine, Volume 47, Issue 2, July 1995, Pages 131 -146), described in WO2022/019293 (PTL 2). The conditions and results of MCML are explained as follows. A three-layer structure consisting of a skin layer, a fat layer, and a muscle layer was modeled, and the optical characteristics of each were set as follows. Note that the values are for skin, fat, and muscle, respectively (Phys. Med. Biol. 44 (1999) 2689-2702).
Refractive index n: 1.4 for all
Absorption coefficient µa: 0.15 cm⁻¹, 0.02 cm⁻¹, 0.3 cm⁻¹
Scattering coefficient µs: 100 cm⁻¹, 80 cm⁻¹, 33 cm⁻¹
Isotropic scattering parameter g: 0.85 for all
Thickness: 0.1 cm, 0.4 cm, 2.0 cm

Under the muscle layer, it is assumed that a layer having a refractive index of 1.4 continues infinitely. The light beam irradiation conditions were assumed as follows: beam profile of Gaussian spatial intensity distribution, irradiation radius of 0.9 cm, and energy of 600 J. The number of photons was set to 10 million, and calculations were performed in increments of 0.05 cm in depth and radial directions.

The results are explained as follows. The average power density at a depth of 15 mm, which corresponds to the depth of the vagus nerve in guinea pigs, was 6.1 mW/cm², and the average power density at a depth of 10 mm in humans was 20.3 mW/cm². Therefore, in order to reproduce in a human the average power density at the vagus nerve depth of a guinea pig, the optimal clinically equivalent irradiation conditions can be derived by multiplying conditions related to the light dose in a guinea pig such as peak power, average power, energy, and energy density by 0.3 (the average power density at the vagus nerve depth of a guinea pig/that of a human).

### [Example 2] Efficacy evaluation test using a cough model by light beam irradiation

To investigate the effectiveness of low-level laser therapy (LLLT) on cough, we conducted a study using an animal cough model. As a cough model, we used the citric acid-induced cough model in guinea pigs, which is widely used in non-clinical studies of cough, and in which cough is induced by activating the vagus nerve.

The test procedure is as follows.

### Animal model used

Used was the citric acid-induced cough model in guinea pigs, in which cough is induced by stimulating Aδ and C fibers (TRPV1) with citric acid, and used were male guinea pigs, Slc:Harltley, 6 weeks old. The groups were set as follows: 1: healthy group, 2: laser sham irradiation group, 3: laser irradiation group, and 4: positive control group, with eight mice in each group. The no laser irradiation group was a group in which the same experimental procedure as laser irradiation was performed without laser output, and the positive control group was a group administered with an antitussive drug of dihydrocodeine phosphate.

Prior to the test, the location of the cervical vagus nerve ganglion of the guinea pigs was confirmed by dissection. Behind the masseter muscle, confirmed was a ganglion where the vagus nerve and superior laryngeal nerve both causing cough intersected with each other. The ganglion was confirmed to be at a depth of approximately 15 mm from the epidermis, the depth of which sufficiently allows arrival of a nerve-suppressing laser beam.

### Laser irradiation conditions

A laser beam percutaneous irradiation was performed at a position near the masseter muscle of the guinea pig under the following irradiation conditions. Wavelength: 808 nm, average output: 0.75 W, peak output: 7.5 W; pulse oscillation, frequency: 5 Hz, duty ratio: 10%, irradiation area: 0.8 cm², irradiation time: 5 minutes each on the left and right.

### Evaluation method

The effectiveness of cough treatment was evaluated by visually counting the number of coughs and comparing the number of coughs after laser irradiation or antitussive drug administration (Post).

### Experimental techniques

(1) The animals were placed in a chamber for cough detection, and citric acid was atomized using an ultrasonic nebulizer for the animals to inhale for 10 minutes, while visually counting the number of coughs (pre-measurement).
(2) After the pre-measurement, the subjects were divided into groups equally based on the number of cough reflexes.
(3) Thirty minutes before citric acid inhalation (approximately three hours after the pre-measurement), laser irradiation was performed for the laser irradiation group on the cervical vagus nerve, and dihydrocodeine phosphate was administered to the positive control group.
(4) The animals were placed again in the cough detection chamber, and citric acid was atomized using an ultrasonic nebulizer for the animals to inhale for 10 minutes, while visually counting the number of coughs (post-measurement).
(5) The effectiveness of laser irradiation was evaluated based on the number of cough reflexes in the post-measurement. Dunnett's multiple comparison test was performed between the sham group and all groups, with a significance level of 5%.

### Result

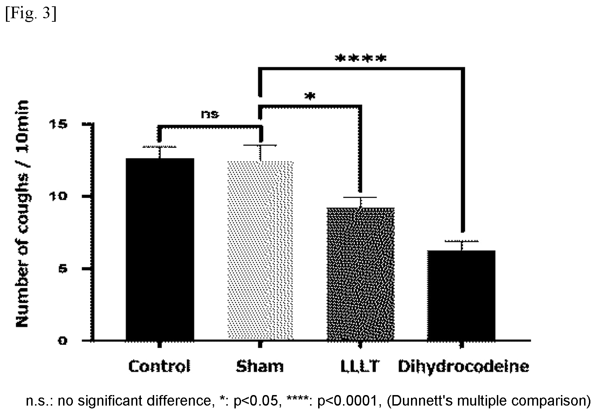

The evaluation results of the effectiveness of LLLT in treating cough are shown in Fig. 3 and Table 1 below.

**[Table 1]**

| Group | Number of samples | Average | Standard deviation | Test result vs. Sham |
|---|---|---|---|---|
| Control | 8 | 12.6 | 0.8 | 0.9990 |
| Sham | 8 | 12.5 | 1.1 | - |
| LLLT | 8 | 9.3 | 0.7 | 0.0223 |
| Dihydrocodeine | 8 | 6.3 | 0.6 | <0.0001 |

No significant difference was confirmed between the sham laser irradiation group (Sham group) and the healthy group (Control group), and significant reduction in the number of coughs was confirmed between the Sham group and the positive control group (Dihydrocodeine group), and between the Sham group and the laser irradiation group (LLLT group).

The LLLT group showed a smaller reduction in the number of coughs than the dihydrocodeine group, which was presumably caused by the influence of the evaluation time and the misalignment of irradiation position. Similarly to the centrally acting antitussive of dihydrocodeine, low-power laser irradiation on the cervical vagus nerve was confirmed to significantly reduce the number of coughs.

### [Industrial Applicability]

There can be provided a new physical treatment method, which has confirmed cough suppression effect by phototherapy, as a new treatment method for cough.

### [Reference Signs List]

1 Light beam irradiation device
2 Light beam irradiation probe
3 Light beam source
4 Probe cable
5 Main body
6 Light beam source and power supply

## Claims

1. A cough treatment device, comprising: a light beam source emitting a light beam and a light beam irradiation probe performing irradiation with the light beam, light beam irradiation being performed percutaneously with the light beam emitted by the light beam source from the light beam irradiation probe toward a patient's cervical vagus nerve and the vicinity thereof.

2. The cough treatment device according to claim 1, wherein the light beam irradiation probe is a device that performs irradiation toward an inferior ganglion, superior ganglion, pharyngeal branch, or superior laryngeal nerve of a cervical vagus nerve.

3. The cough treatment device according to claim 1, wherein the light beam irradiation probe is a device that irradiates an area behind an angle of mandible or below a mastoid process in the patient's neck.

4. A cough treatment device according to claim 1, wherein an average power of a light beam of irradiation from the light beam irradiation probe is 200 mW to 8 W, an average power density obtained by dividing an average power of a light beam of irradiation from the light beam irradiation probe by the irradiation area of the light beam is 250 mW/cm² or more to 10,000 mW/cm² , an energy of the amount of light beam irradiation from the light beam irradiation probe is 60 J to 4,800 J per treatment, an energy density of the amount of light beam irradiation from the light beam irradiation probe divided by the irradiation area of the light beam is 75 J/cm² to 6,000 J/cm² per treatment, and a wavelength of a light beam of irradiation from the light beam irradiation probe is 700 nm to 900 nm.
